(19) Europäisches Patentamt
European Patent Office
Office européen des brevets

(11) Veröffentlichungsnummer: **0 551 642 A1**

(12) # EUROPÄISCHE PATENTANMELDUNG

(21) Anmeldenummer: **92121772.5**

(51) Int. Cl.[5]: **C07D 487/04**

(22) Anmeldetag: **22.12.92**

(30) Priorität: **16.01.92 DE 4200933**

(43) Veröffentlichungstag der Anmeldung: **21.07.93 Patentblatt 93/29**

(84) Benannte Vertragsstaaten: **AT BE CH DE DK ES FR GB IT LI NL SE**

(71) Anmelder: **BASF Aktiengesellschaft**
**Carl-Bosch-Strasse 38**
**W-6700 Ludwigshafen(DE)**

(72) Erfinder: **Brunner, Henri, Dr.**
**Eichendorffstrasse 14**
**W-8417 Lappersdorf(DE)**
Erfinder: **Huber, Christian, Dr.**
**Adlerstrasse 19b**
**W-8440 Straubing(DE)**
Erfinder: **Bublak, Petra**
**Landgraf-Ulrich-Strasse 21**
**W-8473 Pfreimd(DE)**

(54) **Verfahren zur diastereoselektiven Hydrierung von Folsäure zu Tetrahydrofolsäure.**

(57) Verfahren zur diastereoselektiven Hydrierung von Folsäure zu Tetrahydrofolsäure, das dadurch gekennzeichnet ist, daß man die Hydrierung der Folsäure in Gegenwart eines immobilisierten Rhodium(I)-Komplexes eines optisch aktiven Diphosphins in einer neutralen wäßrigen Pufferlösung bei einem $H_2$-Druck von mehr als 20 bar und einer Temperatur von mehr als 60 °C durchführt.

EP 0 551 642 A1

Die Erfindung betrifft ein Verfahren zur diastereoselektiven Hydrierung von Folsäure zu Tetrahydrofolsäure in Gegenwart eines chiralen, immobilisierten Rhodium(I)/Diphosphin-Katalysators.

Folsäure (1), ein wasserlösliches Vitamin, und Biopterin sind C(6)-substituierte Pterin-Derivate. Während die Tetrahydro-Form des Biopterins zur Behandlung der Parkinson'schen Krankheit von großem Interesse ist, spielen die verschiedenen Tetrahydrofolate eine herausragende Rolle als Coenzyme bei der Übertragung von Methylgruppen auf heterocyclische Vorstufen von Nucleinbasen in der DNA-Synthese.

Die unreduzierte Form der Folate zeigt noch keine biologische Aktivität bei der Übertragung von Einkohlenstoff-Fragmenten. Der eigentliche "Carrier" für Einkohlenstoffeinheiten ist die 5,6,7,8-Tetrahydrofolsäure (2a). Bei der enzymatischen Reduktion der Folsäure bildet sich zunächst die 7,8-Dihydrofolsäure, die vom Enzym Dihydrofolat-Reduktase in die physiologisch wirksame Tetrahydro-Form überführt wird.

Schnell proliferierende Krebszellen benötigen zum Aufbau ihrer DNA in verstärktem Ausmaß Nucleinbasen. Diese Eigenschaft macht man sich bei der chemotherapeutischen Behandlung verschiedener Krebsarten zunutze. Folat-Antagonisten wie Methotrexat hemmen die Dihydrofolat-Reduktase durch eine pseudo-irreversible Verdrängung der natürlichen Substrate. Durch diese Enzyminhibierung wird die Versorgung der Zellen mit DNA-Bausteinen unterbrochen, und es kommt zum Zelltod.

Bei Verabreichung hoher Dosen Methotrexat muß deshalb ein sogenanntes "rescue agent" eingesetzt werden, um die normalen Körper- und Zellfunktionen aufrecht zu erhalten. Zur Behebung eines bei der Methotrexat-Therapie verursachten akuten Folsäuremangels wird das Calciumsalz der Folinsäure (2b bzw. 3b) appliziert, auch Leucovorin oder Citrovorum-Faktor (Wachstumsfaktor des Bakteriums Leuconostoc citrovorum) genannt. Durch diese in der N(5)-Position formylierte 5,6,7,8-Tetrahydrofolsäure kommt es zu einem Wiedereinsetzen des Folatmetabolismus. Darüber hinaus wird Leucovorin zusammen mit 5-Fluoruracil in Kombinationspräparaten gegen fortgeschrittene Colonkrebse eingesetzt. Diese Anwendung spielt mengenmäßig sogar eine größere Rolle als die Anwendung als Antidot gegen hochdosiertes Methotrexat bei Osteosarkomen. Zunehmende klinische Bedeutung hat das Medikament Leucovorin auch bei der Behandlung der megaloblastischen Folsäuremangel-Anämie, der rheumatischen Arthritis und zur Verbesserung der Verträglichkeit mancher Antibiotikapräparate. Eine mindestens gleich gute Wirkung wie Leucovorin hat auch das neuerdings im Handel befindliche Calcium-5-Methyl-tetrahydrofolat.

Bei den zur Zeit im technischen Maßstab nutzbaren Leucovorin-Synthesen (vgl. W. Pfleiderer, Comprehensive Heterocyclic Chemistry 3 (2B), 305 (1984)) wird die Folsäure mit Hilfe von Platinoxid oder Palladium auf einem Trägermaterial katalytisch hydriert. Diese Reaktionen werden wegen der Schwerlöslichkeit der Folsäure in stark polaren Lösungsmitteln wie Eisessig, Trifluoressigsäure und Ameisensäure durchgeführt. Auch die Reduktion mit Natriumboranat in schwach alkalischer, wäßriger Lösung ist möglich. Bei der Reduktion der prochiralen C=N-Doppelbindung des Pyrazinrings der Folsäure bildet sich ein neues Asymmetriezentrum am C(6)-Atom des Pterinsystems. Da die Folsäure die (S)-konfigurierte Glutaminsäure enthält, weisen die beiden sich bildenden Diastereomeren der Tetrahydrofolsäure (2a bzw. 3a) (6S,S)- bzw. (6R,S)-Konfiguration auf. Die chirale Information im Glutaminsäure-Molekülteil entfaltet keine nennenswerte optische Induktion, so daß die beiden Diastereomeren im Verhältnis von etwa 1:1 anfallen. Das aus der Tetrahydrofolsäure dargestellte Medikament Leucovorin wird bis heute als 1:1-Gemisch der Diastereomeren 2b und 3b eingesetzt.

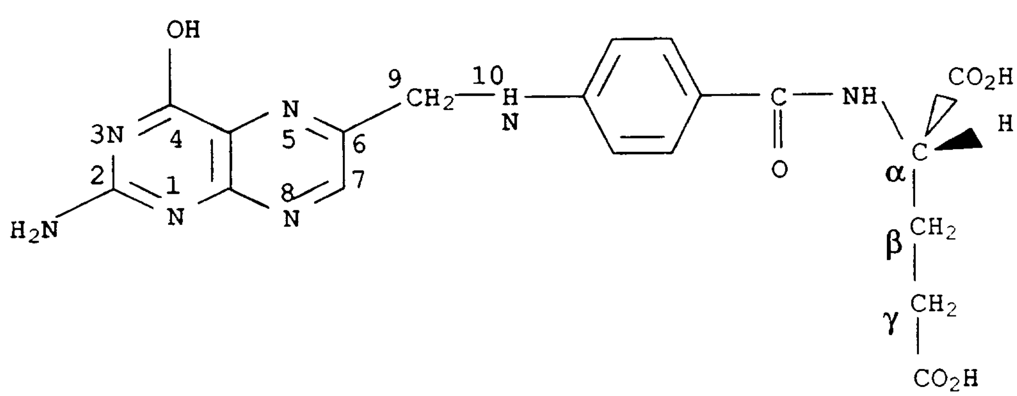

Folsäure (1)

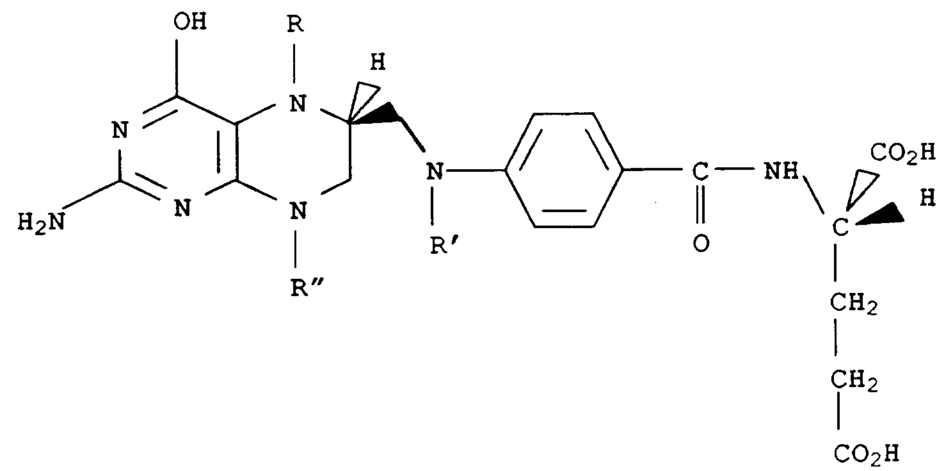

2a) (6S,S)-Tetrahydrofolsäure (R = R' = R'' = H)
2b) (6S,S)-Leucovorin (R = -CHO; R' = R'' = H)
2c) (6S,S)-Derivat von 2a) mit (-)-Chlorameisensäurementhylester (R = -COOMenthyl; R' = R'' = H)

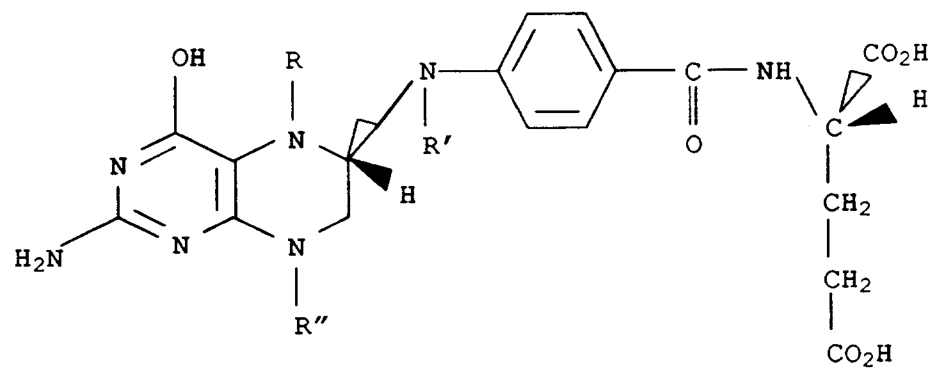

3a) (6R,S)-Tetrahydrofolsäure (R = R' = R'' = H)
3b) (6R,S)-Leucovorin (R = -CHO; R' = R'' = H)
3c) (6R,S) -Derivat von 2a) mit (-)-Chlorameisensäurementhylester (R = -COOMenthyl; R' = R'' = H)

Nur das natürlich vorkommende (6S,S)-Diastereomere 2b dient im Zellmetabolismus als Carrier-System für Einkohlenstoffgruppen, wohingegen das (6R,S)-Isomere 3b keinerlei Aktivität aufweist und sogar als Enzyminhibitor fungiert (vgl. C.J. Temple et al., Cancer Treat. Rep. 65, 1117 (1981); G.K. Smith et al., Biochemistry 20, 4034 (1981); R.P. Leary et al., Biochem. Biophys. Res. Commun. 56, 484 (1973); V.F. Scott, ibid. 14, 523 (1964); J.A. Straw et al.: Cancer Res. 41, 3936 (1981) und 44, 3114 (1984); R. Bertrand et al. J. Nat. Cancer Inst. 81, 1175 (1989)). Neuere Arbeiten zeigen, daß die (6R,S)-Form langsamer metabolisiert und ausgeschieden wird als die (6S,S)-Form. Durch die Applikation eines Gemisches der Diastereomeren 2b und 3b kommt es hauptsächlich im zentralen Nervensystem zu einer Anreicherung der im Metabolismus gebildeten Tetrahydrofolate mit der unnatürlichen (6R,S)-Konfiguration 3 und damit zu einer Intoxikation.

Im Laufe der letzten 30 Jahre sind daher große Anstrengungen unternommen worden, das wirksame (6S,S)-Isomere gezielt herzustellen. Folgende Möglichkeiten zur Abtrennung des (6S,S)-Isomeren vom (6R,S)-Isomeren wurden bisher beschrieben:

a) Fraktionierte Kristallisation der Erdalkalisalze der Folinsäure 2b und 3b (vgl. D.B. Cosulich et al., J. Am. Chem. Soc. 74, 3252 (1952) und 75, 4215 (1953); US 2 688 018; EP 88/00341),

b) Lösungsmittelextraktion des Isomerengemisches von 5-Menthyloxycarboxyl-tetrahydrofolsäure (vgl. L. Rees et al. J. Chem. Soc. Chem. Comm. 470 (1987); GB 8 621 268; EP 266 042) und

c) Präparative Chromatographie der Folinsäure 2b und 3b (vgl. J. Feeney et al., Biochemistry 20, 1837 (1981); C.K. Mathews et al. J. Biol. Chem. 235, 3304 (1969)).

Ein gemeinsamer Nachteil dieser drei Verfahren ist die Tatsache, daß von einem 1:1-Gemisch der Isomeren ausgegangen wird. Die Isomerentrennung führt also immer zu einem Substanzverlust von mindestens 50 %.

In jüngster Zeit wurde darüberhinaus ein biotechnologisches Verfahren zur Herstellung von (6S,S)-Tetrahydrofolsäure durch Behandeln von Dihydrofolsäure mit Dihydrofolat-Reduktase bekannt (vgl. EP-A2 356 934). Nachteilig an diesem Verfahren ist, daß die als Ausgangsverbindung benötigte Dihydrofolsäure in einer gesonderten Reaktionsstufe aus Folsäure hergestellt werden muß.

Es war daher die Aufgabe der Erfindung ein Verfahren zu entwickeln, mit dessen Hilfe man Folsäure oder Derivate davon möglichst diastereoselektiv direkt zu den entsprechenden Tetrahydroverbindungen mit (6S,S)-Konfiguration hydrieren kann.

Gegenstand der Erfindung ist ein Verfahren zu diastereoselektiven Hydrierung von Folsäure, das dadurch gekennzeichnet ist, daß man die Hydrierung der Folsäure in Gegenwart eines immobilisierten Komplexes aus einer Rhodium(I)-Verbindung und einem optisch aktiven Diphosphin in einer wäßrigen Pufferlösung mit einem pH-Wert von 3 bis 12, vorzugsweise einem pH-Wert von etwa 7, bei einem $H_2$-Druck von mehr als 20 bar, vorzugsweise mindestens 30 bar und einer Temperatur von mehr als 60 °C, vorzugsweise 70 bis 90 °C, insbesondere etwa 80 °C, durchführt.

Es sind zwar schon zahlreiche Verfahren zur enantioselektiven Hydrierung von Verbindungen, die eine C = C-Doppelbindung, oder gar eine C = N-Doppelbindung tragen, an Rhodium(I)-Komplexen von optisch aktiven Diphosphinen bekannt, jedoch war nicht zu erwarten, daß man auch Bedingungen finden könnte, unter denen auch die in hohem Grade instabile und zersetzliche Tetrahydrofolsäure herstellbar ist.

Folsäure ist eine bekannte und handelsübliche Verbindung, so daß auf deren Herstellung nicht näher eingegangen zu werden braucht.

Der als Katalysator verwendete Komplex muß in immobilisierter Form vorliegen. Diese Immobilisierung erleichtert nicht nur die Aufarbeitung und Recyclisierung, sondern ist für den Erfolg der erfindungsgemäßen Umsetzung zwingend erforderlich.

Ob bei dem erfindungsgemäßen Verfahren das (6S,S) oder (6R,S)-Isomere im Überschuß gebildet wird, hängt von der Konfiguration des für die Komplexbildung verwendeten optisch aktiven Diphosphins ab.

Als optisch aktive organische Diphosphine für die Komplexverbindung können beispielsweise folgende Verbindungen verwendet werden:

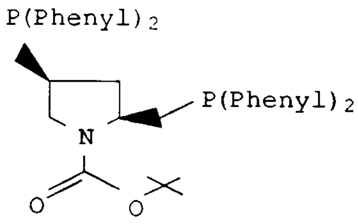

(2S,4S)-N-tert.-Butoxycarbonyl-4-diphenylphosphino-2-diphenylphosphinomethyl-pyrrolidin

((-)-BPPM; vgl. JACS 98 (1976) 8265)

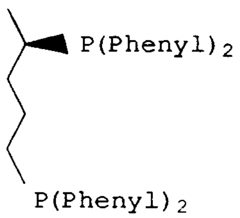

(S)-1,4-Bis(diphenylphosphino)pentan

((-)-1,4-BDPP; vgl. Synthesis 1989, 706)

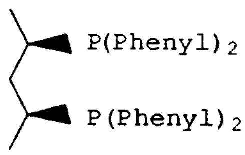

(S,S)-2,4-Bis(diphenylphosphino)pentan

((-)-2,4-BDPP; vgl. J. Organomet. Chem. 279 (1985) 23)

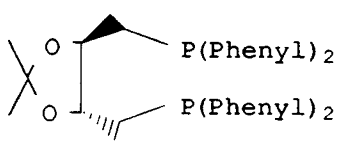

(4R,5R)-2,3-O-Isopropyliden-2,3-dihydroxy-1,4-bis(diphenylphosphino)butan

((-)-DIOP; vgl. JACS 94 (1972) 6429)

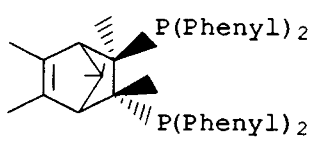

(S,S)-2,3-Bis(diphenylphosphino)-bicyclo[2.2.1]hept-5-en

((+)-NORPHOS; vgl. Angew. Chem. 91 (1979) 655)

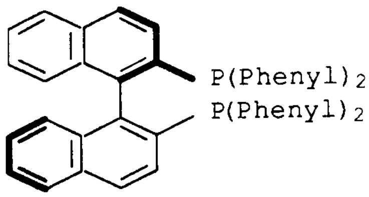

(R)-2,2'-Bis(diphenylphosphino)-1,1'-binaphthyl

((+)-BINAP; vgl. J. Org. Chem. 51 (1986) 629)

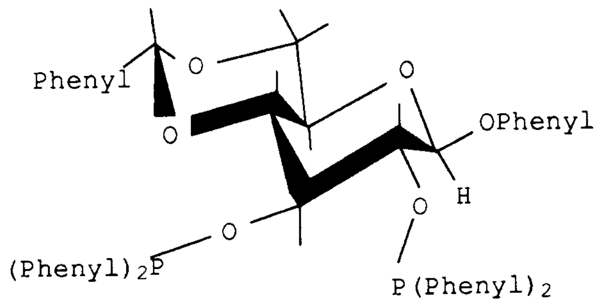

Phenyl-4,6-O-(R)-benzyliden-2,3-O-bis(diphenylphosphino)-ß-D-glucopyranosid

((-)-Ph-ß-glup; vgl. J. Mol. Catal. 37 (1986) 213)

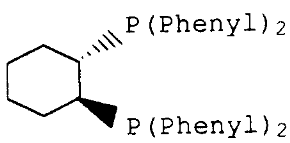

(S,S)-(+)-1,2-Bis(diphenylphosphino)-cyclohexan
(vgl. J. Mol.Catal. 26 (1984) 17)

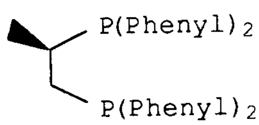

(R)-(+)-1,2-Bis(diphenylphosphino)-propan
((+)-PROPHOS; vgl. JACS 100 (1978) 5491)

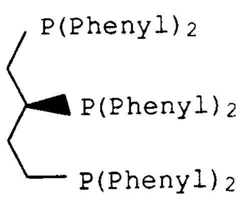

(R)-(+)-1,2,4-Tris(diphenylphosphino)-butan

((+)-1,2,4-TDPB; vgl. Synthesis 1989, 706)

Zur Herstellung der gewünschten (6S,S)-Tetrahydrofolsäure eignen sich beispielsweise immobilisierte Rhodium(I)-Komplexe mit folgenden optisch aktiven Diphosphinen:
(-)-BPPM, (-)-1,4-BDPP, (-)-2,4-BDPP, (-)-DIOP, (+)-NORPHOS, (+)-BINAP und (-)-Ph-$\beta$-glup.

Einen besonders hohen Diastereomerenüberschuß zugunsten der (6S,S)-Tetrahydrofolsäure erzielt man mit (-)-BPPM, (-)-1,4-BDPP, (-)-2,4-BDPP, (-)-DIOP und (+)-NORPHOS, insbesondere (-)-BPPM und (-)-1,4-BDPP.

Bevorzugt (6R,S)-Tetrahydrofolsäure erhält man dagegen mit Rhodium(I)-Komplexen enthaltend beispielsweise folgende optisch aktiven Diphosphine: (+)-PROPHOS, (+)-DIOP, (-)-NORPHOS und (+)-1,3,4-TDPB.

Als Rhodium(I)-Verbindungen, die mit den oben genannten optisch aktiven Diphosphinen einen Komplex bilden können, seien beispielsweise genannt:
$[RhCl(COD)]_2$, worin COD für 1,5-Cyclooctadien steht.

Aber auch Rhodium(I)-Verbindungen, die anstelle von COD Norbornadien, 1,5-Hexadien, Ethylen (2x), Cycloocten (2x) enthalten, können verwendet werden. Auch kationische Rhodium(I)-Verbindungen wie [Rh-

$(COD)_2]^+$ und $[Rh(Norbornadien)_2]^+$ mit den Anionen $BF_4^-$ oder auch $ClO_4^-$ sind geeignet. Mit besonderem Vorteil arbeitet man mit $[RhCl(COD)]_2$.

Die Art der Rhodiumverbindung hat keinen großen Einfluß auf die optische Induktion.

Das Immobilisieren des chiralen Rhodium(I)-Diphosphinkomplexes kann folgendermaßen durchgeführt werden. Man legt $[RhCl(COD)]_2$, das optisch aktive Diphosphin und den Träger vor und gibt Methylenchlorid oder ein anderes organisches Lösungsmittel zu. Das Gemisch wird eine halbe Stunde bei Raumtemperatur gerührt. Anschließend wird das Lösungsmittel abgezogen. Der Komplex, der sich dabei aus $[RhCl(COD)]_2$ und dem optisch aktiven Diphosphin gebildet hat, ist dann auf der Trägeroberfläche fixiert.

Als Trägermaterial können beispielsweise $SiO_2$, wie beispielsweise Kieselgel 60: 70 bis 120 mesh ASTM; (0.125 bis 0.200 mm) von Merck; LiChroprep Si60,Korngröße 15 bis 25 $\mu$m von Merck oder Kieselgel 60: 70 bis 230 mesh ASTM von Merck; $Al_2O_3$, wie $Al_2O_3$ Merck, 70 bis 120 mesh ASTM, Akt. II bis III; $BaSO_4$ oder Cellulose, wie Cellulose Merck CM, verwendet werden. Mit besonderem Vorteil arbeitet man mit einem durch Adsorption an $SiO_2$ immobilisierten Komplex. Hierbei ist die Verwendung von $SiO_2$ mit einheitlicher Korngröße günstiger als die Verwendung von $SiO_2$ mit sehr unterschiedlicher Korngrößenverteilung.

Das optisch aktive Diphosphin setzt man mit der Rhodium(I)-Verbindung im allgemeinen etwa im Verhältnis 1,15:1 zu dem Komplex um.

Zum Immobilisieren verwendet man das Adsorptionsmittel im allgemeinen in Mengen von 70 bis 150, vorzugsweise 100 bis 120 g pro g des Komplexes.

Die bei der Hydrierung verwendete Menge des immoblisierten Katalysatorkomplexes beträgt im allgemeinen 1,0 bis 2,5 Mol-%, vorzugsweise etwa 1,1 bis 1,5 Mol-%, bezogen auf eingesetzte Folsäure.

Die erfindungsgemäße Hydrierung wird mit Vorteil in einer etwa 0,1 molaren Phosphatpufferlösung, die mit einem Alkali- oder Erdalkalihydroxid, vorzugsweise mit NaOH, bei einem pH-Wert von 3 bis 12, vorzugsweise von etwa 7 gehalten wird, durchgeführt. Aber auch ähnliche Pufferlösungen können eingesetzt werden, soweit man mit ihnen den beanspruchten pH-Bereich aufrechterhalten kann.

Zur Durchführung des Verfahrens geht man im allgemeinen so vor, daß man die Folsäure unter Schutzgasatmosphäre in einer neutralen wäßrigen Pufferlösung suspendiert und durch Zugabe von einem Alkali- oder Erdalkalihydroxid unter Aufrechterhalten eines pH-Wert von vorzugsweise etwa 7 löst, dann den immobilisierten Rhodium(I)-Komplex zufügt und anschließend in einem Autoklaven bei einem Wasserstoffdruck von mindestens 20 bar und 60 °C hydriert. Der nach der Hydrierung leicht abfiltrierbare Katalysator kann mehrfach verwendet werden. Bei mehrfacher Verwendung sinkt jedoch bei einigen Katalysatoren der Hydriergrad ab und auch die erzielten Diastereomerenüberschüsse werden geringer.

Die Reaktionszeiten betragen je nach Reaktionstemperatur etwa 15 bis 30, vorzugsweise etwa 20 bis 24 Stunden.

Mit Hilfe des erfindungsgemäßen Verfahrens kann Tetrahydrofolsäure in einem Diastereomerenüberschuß zugunsten der (6S,S)-Tetrahydrofolsäure von bis zu 60 % erhalten werden, entsprechend einem Diastereomerenverhältnis von (6S,S):(6R,S)≈80:20. Ausgehend von der erfindungsgemäß hergestellten Tetrahydrofolsäure können in der Krebstherapie einsetzbare Mittel, wie Leucovorin und Calcium-5-Methyltetrahydrofolat in wesentlich wirksamerer Form hergestellt werden.

## Beispiele

Alle Reaktionen wurden unter Schutz durch ein Inertgas durchgeführt. Die angesetzten Lösungsmittel und Reagenzien wurden vor der Umsetzung von gelöstem Sauerstoff befreit.

A. Immobilisierung der Rhodium(I)/Diphosphin-Komplexe

In einem Autoklaveneinsatz wurden 7 mg (entsprechend 0,028 mmol Rh) an $[Rh(COD)Cl]_2$, 0,033 mmol (-)-BPPM und 0,80 g Kieselgel eingewogen und mit Stickstoff gesättigt. Nach dem Lösen in 10 ml Methylenchlorid schlug die gelbe Farbe nach orange um. Diese zunächst tiefgefärbte Suspension ließ man eine halbe Stunde (h) bei Raumtemperatur (RT) rühren, währenddessen der orange Rhodium(I)-/Diphosphin-Komplex unter Entfärbung der organischen Lösung an der Oberfläche des Trägermaterials adsorbiert wurde. Anschließend wurde bei RT unter vermindertem Druck (Ölpumpe) das organische Lösungsmittel abgezogen und das gelborange Katalysator-Pulver getrocknet. Die Herstellung der Katalysatoren mit anderen Diphosphinen erfolgte ganz analog.

B. Durchführung der heterogen katalysierten Hydrierung

Alle Hydrierungen wurden in einem 100-ml-Laborautoklaven mit Manometer durchgeführt. Jeweils 0,50 g (1,13 mmol) einer mit Stickstoff gesättigten Folsäure wurden in 25 ml eines 0,1 molaren Phosphatpuffers mit einem pH = 7,0 suspendiert und durch Zusetzen einer äquimolaren Menge an Natronlauge gelöst, ohne den neutralen Pufferbereich zu verlassen. Die klare, dunkelorange Lösung wurde anschließend

unter Schutzgasatmosphäre zu dem im Autoklaveneinsatz vorgelegten, immobilisierten Rhodium(I)-Katalysator zugegeben. Der Autoklav wurde dreimal mit 20 bar Wasserstoff gespült und auf den Standarddruck von 45 bar gebracht. Dann ließ man jeden Ansatz 24 h bei 80 °C rühren. Nach dem Abkühlen auf RT wurde die gelbliche Lösung über eine GIII-Filterfritte vom heterogenen Katalysator abgetrennt. Den festen Katalysator-Rückstand wusch man mit 3 ml des entsprechenden Phosphatpuffers. Die instabilen Hydrierprodukte wurden nach zwei Methoden analysiert.

(1) Überführung in Leucovorin und Analytik mit einer BSA-HPLC-Säule:

Die erhaltene Lösung wurde zur Trockne eingeengt. Der Rückstand wurde mit DMSO/Pyridin 5:1 unter Lichtausschluß 72 h mit Methylformiat/Ameisensäure behandelt und dabei in bekannter Weise in Leucovorin überführt. Die Analytik erfolgte mit Hilfe der HPLC-Technik mit einer BSA-Säule in bekannter Weise. Die erhaltenen Hydriergrade sowie die Werte für die Diastereomerenüberschuß (de) sind in der folgenden Tabelle zusammengestellt.

(2) Derivatisierung mit Chlorameisensäurementhylester und Analytik mit einer Reversed-Phase-Säule:

Die Hydrierprodukte wurden mit (-)-Chlorameisensäurementhylester (im Molverhältnis 1:1) derivatisiert. Die erhaltene Derivate wurden auf einer Reversed-Phase-Säule flüssigchromatographisch in bekannter Weise aufgetrennt. Die erhaltenen Hydriergrade sowie die Werte für den Diastereomerenüberschuß (de) sind in der folgenden Tabelle zusammengestellt.

Der abfiltrierte Katalysator wurde 3x mit je 20 mi Sauerstoff-freiem Wasser gewaschen und unter vermindertem Druck (Ölpumpe) mehrere h getrocknet. Das resultierende hellbeige Pulver kann wieder als Hydrierkatalysator verwendet werden.

Tabelle

| Verwendetes Diphosphin | Hydriergrad [%] | Diastereomerenüberschuß de [%] | überwiegende Konfiguration |
|---|---|---|---|
| (-)-BPPM[a),c)] | 100 | 49 - 51 | (6S,S) |
| (-)-BPPM[a),d)] | 100 | 38 | (6S,S) |
| (-)-BPPM[a),e)] | 100 | 40 - 46 | (6S,S) |
| (-)-1,4-BDPP[a),b),e)] | 96 - 100 | 20 - 24 | (6S,S) |
| (-)-2,4-BDPP[b),e)] | 98 - 99 | 18 - 20 | (6S,S) |
| (+)-DIOP[a),b),e)] | 96 - 100 | 10 - 12 | (6R,S) |
| (-)-DIOP[a),b),e)] | 96 - 100 | 18 - 22 | (6S,S) |
| (-)-DIOP[a),e)] | 100 | 29 - 33 | (6S,S) |
| (-)-DIOP[a),c)] | 100 | 53 | (6S,S) |
| (+)-NORPHOS[a),b),e)] | 96- 100 | 16 - 22 | (6S,S) |
| (-)-NORPHOS[a),b),e)] | 98 - 99 | 11 - 14 | (6R,S) |
| (+)-BINAP[b),e)] | 78 - 82 | 15 - 16 | (6S,S) |
| (-)-Ph-β-glup[b),e)] | 81 - 83 | 8 - 12 | (6S,S) |
| (+)-Cyclohexanphos[a),e)] | 100 | 2 - 3 | (6S,S) |
| (+)-PROPHOS[a),e)] | 97 - 100 | 2 - 3 | (6R,S) |
| (+)-1,2,4-TDPB[b),e)] | 85 - 88 | 1 - 3 | (6R,S) |

[a)] Analytik (1)
[b)] Analytik (2)
[c)] Kieselgel 60: 70 bis 120 mesh ASTM, (0,125 bis 0,200 mm) von Merck
[d)] Kieselgel LiChroprep Si60, Korngröße 15 bis 25 µm von Merck
[e)] Kieselgel 60: 70 bis 230 mesh ASTM, Merck.

**Patentansprüche**

1. Verfahren zur diastereoselektiven Hydrierung von Folsäure zu Tetrahydrofolsäure, dadurch gekennzeichnet, daß man die Hydrierung der Folsäure in Gegenwart eines immobilisierten Rhodium(I)-Komplexes eines optisch aktiven organischen Diphosphins in einer wäßrigen Pufferlösung mit einem pH-Wert von 3 bis 12, bei einem $H_2$-Druck von mehr als 20 bar und einer Temperatur von mehr als 60 ° C durchgeführt.

2. Verfahren gemäß Anspruch 1, dadurch gekennzeichnet, daß man die Hydrierung in einer wäßrigen Pufferlösung mit einem pH-Wert von etwa 7 durchführt.

3. Verfahren gemäß Anspruch 1, dadurch gekennzeichnet, daß man die Hydrierung bei einem $H_2$-Druck von mindestens 30 bar durchführt.

4. Verfahren gemäß Anspruch 1, dadurch gekennzeichnet, daß man die Hydrierung bei Temperaturen von 70 bis 90 ° C durchführt.

5. Verfahren gemäß Anspruch 1, dadurch gekennzeichnet, daß man zur Erzielung eines Diastereomerenüberschusses zugunsten der (6S,S)-Tetrahydrofolsäure einen immobilisierten Rhodium(I)-Komplex verwendet, der als optisch aktives Diphosphin (2S,4S)-(-)-N-tert.-Butoxycarbonyl-4-diphenylphosphino-2-diphenylphosphinomethylpyrrolidin, im folgenden mit (-)-BPPM abgekürzt, (S)-(-)-1,4-Bis-(diphenylphosphino)pentan (-)-1,4-BDPP), (S,S)-(-)-2,4-Bis(diphenylphosphino)pentan((-)-2,4-BDPP), (R,R)-(-)-2,3-0-Isopropyliden-2,3-dihydroxy-1,4-bis(diphenylphosphino)butan ((-)-DIOP), (S,S)-( + )-2,3-Bis(diphenylphosphino)bicyclo[2.2.1]hept-5-en (( + )-NORPHOS), (R)-( + )-2,2'-Bis(diphenylphosphino)-1,1'-binaphthyl (( + )-BINAP), (-)-Phenyl-4,6-0-(R)-benzyliden- 2,3-0-bis(diphenylphosphino) $\beta$-D-glucopyranosid ((-)-Ph-$\beta$-glup) oder (S,S)-( + )-1,2-Bis(diphenylphosphino)cyclohexan enthält.

6. Verfahren gemäß Anspruch 1, dadurch gekennzeichnet, daß man zur Erzielung eines Diastereomerenüberschusses zugunsten der (6S,S)-Tetrahydrofolsäure) einen immobilisierten Rhodium(I)-Komplex verwendet, der als optisch aktives Diphosphin(-)-BPPM enthält.

Europäisches Patentamt

# EUROPÄISCHER RECHERCHENBERICHT

Nummer der Anmeldung

EP 92 12 1772
PAGE1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | CHEMICAL ABSTRACTS, vol. 105, no. 25, 22. Dezember 1986, Columbus, Ohio, US; abstract no. 226387e, * Zusammenfassung * & US-A-748 854 (UNITED STATES DEPT. OF HEALTH AND HUMAN SERVICES) Appl. 26. Juni 1985 --- | 1-6 | C07D487/04 |
| A | CHEMICAL ABSTRACTS, vol. 111, no. 21, 20. November 1989, Columbus, Ohio, US; abstract no. 193822m, PAVLOV V.A. ET AL. * Zusammenfassung * & J. MOL. CATAL. Bd. 44, Nr. 2, 1988, Seiten 217 - 243 --- | 1-6 | |
| A | CHEMICAL ABSTRACTS, vol. 112, no. 19, 7. Mai 1990, Columbus, Ohio, US; abstract no. 177989m, FAHRANG R. ET AL. * Zusammenfassung * & BULL. SOC. CHIM. BELG. Bd. 98, Nr. 6, 1989, Seiten 387 - 393 --- | 1-6 | RECHERCHIERTE SACHGEBIETE (Int. Cl.5)<br>C07D |
| A | CHEMICAL ABSTRACTS, vol. 113, no. 21, 19. November 1980, Columbus, Ohio, US; abstract no. 190720g, WANG H. ET AL. * Zusammenfassung * & CHEM. LETT. Bd. 8, 1990, Seiten 1331 - 1334 --- -/-- | 1-6 | |

Der vorliegende Recherchenbericht wurde für alle Patentansprüche erstellt

| Recherchenort | Abschlußdatum der Recherche | Prüfer |
|---|---|---|
| BERLIN | 23 MAERZ 1993 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 12 1772
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF ORGANIC CHEMISTRY<br>Bd. 45, Nr. 23, 7. November 1980, EASTON US<br>Seiten 4680 - 4682<br>MEYER D. ET AL.<br>--- | 1-6 | |
| A,P | CHEMISCHE BERICHTE<br>Bd. 125, Nr. 9, September 1992, WEINHEIM DE<br>Seiten 2085 - 2093<br>BRUNNER H. ET AL.<br>----- | 1-6 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 23 MAERZ 1993 | FRELON D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument